# EUROPÄISCHE PATENTANMELDUNG

(11) **EP 4 461 228 A1**
(43) Veröffentlichungstag der Anmeldung: **13.11.2024**
(21) Anmeldenummer: 23197988.1
(22) Anmeldetag: 18.09.2023
(51) Int. Cl.: A61B 6/03, A61B 6/00, G01N 23/046, G21K 1/02

(54) **VERFAHREN ZUM BESTIMMEN EINER STREUSTRAHLVERTEILUNG FÜR EINEN COMPUTERTOMOGRAPHEN**

(71) Anmelder: Siemens Healthineers AG, 91301 Forchheim (DE)
(72) Erfinder: Dr. Flohr, Thomas, 91486 Uehlfeld (DE); Dr. Petersilka, Martin, 91325 Adelsdorf (DE); Dr. Stierstorfer, Karl, 91052 Erlangen (DE)
(74) Vertreter: Siemens Healthineers Patent Attorneys

(57) **Zusammenfassung**

Die vorliegende Erfindung stellt ein Verfahren und ein Computertomographiegerät (1) bereit, um eine Streustrahlverteilung bei einem Objekt (3) zu messen, welches in dem Computertomographiegerät (1) mit einem stationären Detektorring (7) und einem stationären Strahlerring (4) angeordnet ist. Der Detektorring (7) umfasst eine Vielzahl von Detektoren (8) und der Strahlerring (4) umfasst eine Vielzahl von Strahlern (5). Das Verfahren umfasst die folgenden Schritte:
- Sequenzielle Aktivierung (101) von jeweils einem oder jeweils zwei Strahlern (5) aus einer Untergruppe der Vielzahl von Strahlern (6) des Strahlerrings (4), zur Erzeugung von jeweils einem oder jeweils zwei Strahlenfächern (9) aus Röntgenstrahlung,
- Erzeugung eines Absorberschattens (102) innerhalb des jeweils erzeugten Strahlenfächers (9) durch zumindest einen im Strahlenfächer (9) angeordneten Absorber (10),
- Detektion der Röntgenstrahlung (103) im Absorberschatten (11) durch die jeweils im Absorberschatten (11) angeordneten Detektoren (8) des Detektorrings (7) und dadurch Erfassung von Messwerten, und
- Berechnung der Streustrahlverteilung (104) auf Basis der erfassten Messwerte.

## Beschreibung

Die vorliegende Erfindung umfasst ein Verfahren zum Bestimmen einer Streustrahlverteilung für einen Computertomographen, sowie ein Computertomographiegerät.

Unabhängig vom grammatikalischen Geschlecht eines bestimmten Begriffs sind Personen mit männlicher, weiblicher oder andere Geschlechteridentität mit umfasst.

Die Computertomographie (CT) ist ein bildgebendes Verfahren, welches mittels eines Computers aus Absorptionswerten von Röntgenstrahlen, die aus verschiedenen Richtungen durch einen Körper treten, Schnittbilder berechnet. Durch überlagerungsfreie Darstellungen von Körperstrukturen ist das CT-Verfahren unabdingbar in der medizinischen Diagnostik geworden. Deshalb wird daran gearbeitet, dieses Verfahren effizienter, genauer und mit einer reduzierten Strahlenbelastung für den Patienten zu gestalten. Ungenauigkeiten hinsichtlich einer realitätsgetreuen Abbildung können dadurch entstehen, dass gemessene Absorptionswerte durch individuelle Streustrahlung des Patienten überdeckt werden können. Diese Überdeckungen können sich als Artefakte auf dem errechneten Bild widerspiegeln, also zu unerwünschten Abdunkelungen und Aufhellungen führen. Um Streustrahlung zu reduzieren, können zwischen den einzelnen Detektorelementen sogenannte Kollimatoren, z.B. absorbierende Bleche, eingesetzt werden. Allerdings eignen sich Kollimatoren nur für CT-Geräte beispielsweise der 3. Generation, wobei die Röntgenquelle und eine von ihr bestrahlte Detektoreinheit gemeinsam um einen zu untersuchenden Körper rotieren. Für statische CT-Geräte ist eine derartige Problemlösung nicht anwendbar, da ein Detektorelement mit einem Kollimator nur von direkt gegenüberliegenden und nicht von einer seitlichen Position bestrahlbar sein würde. Anders ausgedrückt bestünde keine Möglichkeit einen Detektor aus verschiedene Projektionswinkeln mit Röntgenstrahlung zu bestrahlen, wie es beim statischen CT vorgesehen ist.

Aus der US5125012A ist eine CT-Vorrichtung mit einer stationären, ringförmigen Röntgenquelle und ein in Längsrichtung des Untersuchungsobjektes leicht versetzter stationärer Detektorring bekannt. Das Untersuchungsobjekt wird von einem fächerförmigen Röntgenstrahl von verschiedenen Winkeln aus durchdrungen.

Der Erfindung liegt die Aufgabe zugrunde, ein Verfahren und eine Vorrichtung bereitzustellen, welche eine effiziente Bestimmung der Streustrahlverteilung während einer Messung einer Probe durch ein CT-Gerät umsetzen.

Die Erfindung löst die Aufgabe mit einem Verfahren gemäß Anspruch 1 und einer Vorrichtung gemäß Anspruch 13.

Gemäß einem Aspekt der vorliegenden Erfindung wird ein Verfahren zum Bestimmen einer Streustrahlverteilung bei einem Objekt bereitgestellt, welches in einem Computertomographiegerät (CT-Gerät) mit einem stationären Detektorring und einem stationären Strahlerring angeordnet ist, wobei der Detektorring eine Vielzahl von Detektoren und der Strahlerring eine Vielzahl von Strahlern umfasst, umfassend die Schritte sequenzielle Aktivierung von jeweils einem oder zwei Strahlern aus einer Untergruppe der Vielzahl von Strahlern des Strahlerrings, zur Erzeugung von jeweils einem oder zwei Strahlenfächern aus Röntgenstrahlung, Erzeugung eines Absorberschattens innerhalb des jeweils erzeugten Strahlenfächers durch zumindest einen im Strahlenfächer angeordneten Absorber, Detektion der Röntgenstrahlung im Absorberschatten durch die jeweils im Absorberschatten angeordneten Detektoren des Detektorrings und dadurch Erfassung von Messwerten, Berechnung der Streustrahlverteilung auf Basis der erfassten Messwerte.

Das beanspruchte Verfahren und die beanspruchte Vorrichtung bieten unter anderem den Vorteil, Streustrahlwerte an ausgewählten Positionen während einer Computertomographie des betreffenden Körpers direkt zu messen und daraus eine patientenspezifische Streustrahlverteilung zu bestimmen. Gegenüber reinen Berechnungsmethoden kann hierdurch je untersuchten Körper eine individuelle und angepasste Streustrahlverteilung ermittelt werden. Wenn die Streustrahlverteilung bekannt ist, können die bei der CT-Aufnahme gemessenen Detektionswerte durch Subtraktion der durch Streustrahlung erzeugte Strahlungsintensität korrigiert werden, wodurch die Realitätstreue des CT-Bildes gesteigert werden kann.

Das erfindungsgemäße Verfahren dient zur Bestimmung der individuellen Streustrahlung innerhalb des CT vor oder während einer Aufnahme, z.B. einer diagnostischen CT-Untersuchung. Mit Streustrahlung ist insbesondere Röntgenstrahlung gemeint, die im Objekt/Patienten gestreut, gebeugt oder gebrochen wurde und somit ihre Ausbreitungsrichtung, Strahlstärke, Wellenlänge und/oder Frequenz geändert hat. Die von den Detektoren gemessene Streustrahlung wird einer falschen Projektionsrichtung zugeordnet und führt zu Artefakten im Bild. Mit anderen Worten kommt es durch Streustrahlung zu einer Abweichung von einer wahrheitsgemäßen Darstellung eines Objekts. Streustrahlung wird in der Literatur auch als Diffusstrahlung oder diffuse Strahlung bezeichnet.

Das Objekt im Sinne der Erfindung kann jedes Objekt oder Lebewesen sein, an dem eine CT-Untersuchung durchgeführt werden soll, z.B. ein Mensch oder Tier, insbesondere ein Patient. Des Weiteren kann auch ein Gegenstand, z.B. ein Messphantom oder ein Bauteil ein Objekt sein.

Das CT-Gerät der vorliegenden Erfindung ist insbesondere ein statisches CT-Gerät. Das bedeutet, dass sowohl der stationäre Detektorring als auch der stationäre Strahlerring nicht beweglich, insbesondere nicht relativ zueinander drehbar, angeordnet sind. Folglich bleiben Relativpositionen zwischen einem der Detektoren und einem der Strahler konstant. Der Detektorring und der Strahlerring sind insbesondere zueinander konzentrisch. Ferner erstreckt sich eine Längsachse durch und über die Mittelpunkte des kreisförmigen Detektor- respektive kreisförmigen Strahlerrings hinaus. Sowohl Strahlerring als auch Detektorring können innerhalb eines Gehäuses des CT-Gerätes angeordnet sein. Die Absorber sind vorzugsweise innerhalb des Gehäuses angeordnet, insbesondere unterhalb einer Abdeckung des Gehäuses des Strahler- und Detektorrings. Die Absorber sind gemäß einer bevorzugten Ausführungsform fest im CT-Gerät verbaut, sie können jedoch auch entnehmbar gestaltet sein. Entlang der Längsachse können der Detektorring und der Strahlerring zueinander beabstandet sein. Dadurch kann Röntgenstrahlung, ausgehend vom Strahlerring, erst durch das Objekt und dann auf den Detektorring treffen. An dem Detektorring ist eine Vielzahl von Detektoren angeordnet. Der Strahlerring umfasst eine Vielzahl von Strahlern, welche dazu ausgestaltet sind, unabhängig voneinander Röntgenstrahlung gesteuert zu emittieren. Dadurch können der Ursprung von Röntgenstrahlung und somit der Projektionswinkel steuerbar sein. In bevorzugten Ausführungsformen umfasst jeder Strahler eine dazugehörige Kathode, welche steuerbar ist. Von der steuerbaren Kathode werden Elektronen emittiert und hin zu einer Anode unter einer Spannung beschleunigt, welche unter Aussendung einer Bremsstrahlung bzw. Röntgenstrahlung an der Anode abgebremst werden.

Die Strahler sind insbesondere zueinander winkelversetzt und vorzugsweise gleichmäßig über den Umfang des Strahlerrings hinweg verteilt angeordnet. Gleichmäßig verteilt kann bedeuten, dass die Abstände zwischen einem Strahler und dem jeweils benachbarten Strahler konstant sind. Von der Vielzahl von Strahlern wird eine Untergruppe gebildet, wobei die Strahler der Untergruppe zur Ermittlung der Streustrahlverteilung verwendet werden, aber vorzugsweise auch zur Erfassung von Daten für das CT-Bildes beitragen. Die Strahler der Untergruppe unterscheiden sich nicht von den anderen Strahlern des Strahlerrings, vorzugsweise wird das erfindungsgemäße Verfahren sogar im Rahmen einer normalen CT-Untersuchung durchgeführt. Die Erfindung zeichnet sich durch eine besondere Auswertung der detektierten Messwerte in den Absorberschatten aus. Die Strahler der Untergruppe sind dazu ausgestaltet, sequentiell aktiviert zu werden. Das heißt, dass ein Strahler der Untergruppe nach dem anderen, maximal zwei Strahler gleichzeitig, Röntgenstrahlung emittiert. Dadurch kann eine Zuordnung von Messwerten zu dem jeweiligen Strahler, insbesondere von Messwert zu Projektionswinkel des Strahlers, erfolgen. Bei einer Aktivierung eines Strahlers emittiert dieser Röntgenstrahlung in einem Röntgenfächer, welcher ausgehend von dem Strahler in einer Ebene parallel zu dem Detektorring oder Strahlerring radial expandiert. Der Winkel eines Röntgenstrahls innerhalb des Röntgenfächers wird als Fächerwinkel bezeichnet. Die Reihenfolge der Aktivierung der einzelnen Strahler kann variiert werden.

Ein Absorber im Sinne der Erfindung ist nicht Teil des zu untersuchenden Objekts, sondern ist separat davon angeordnet, insbesondere zusätzlich und ggf. speziell zu Zwecken der Streustrahlbestimmung in das CT-Gerät eingebracht. Ein Absorberschatten entsteht dadurch, dass sich während des Verfahrens ein oder mehrere Absorber im Röntgenfächer befinden, welche die Röntgenstrahlung stark abschwächen, vorzugsweise vollständig absorbieren. Unter einer vollständigen Absorption ist insbesondere eine Aufnahme ohne weitere Reflektion oder Emission der Röntgenstrahlung zu verstehen. In dem Absorberschatten sind Detektoren des Detektorrings angeordnet. Durch die Geometrie des Absorbers ist die räumliche Ausdehnung des Absorberschattens vorbestimmt. Es können ein oder mehrere Detektoren innerhalb eines Absorberschattens liegen.

Die Erfindung geht von der Annahme aus, dass ein Detektor innerhalb eines vollständigen Absorberschattens keine Röntgenstrahlung detektieren könnte, wenn es keine Streustrahlung gäbe. Jedoch haben Versuche gezeigt, dass Detektoren selbst innerhalb des Absorberschattens eine Röntgenstrahlung messen. Folglich muss es sich bei der detektierten Strahlung innerhalb des Absorberschattens zwangsläufig um Streustrahlung handeln. Bei dem erfindungsgemäßen Verfahren wird also die Streustrahlung ausgehend von einigen ausgewählten Winkelpositionen der Untergruppe von Strahlern gemessen und daraus eine Streustahlverteilung geschätzt. Durch die Verwendung von mehreren über den Ring verteilten Strahlern zur Streustrahlmessung erhält man Aufschluss über den Verlauf der Streustrahlung als Funktion der Winkelposition des jeweiligen Strahlers, die auch als Projektionswinkel (α) bezeichnet wird. Darüber hinaus kann die Streustrahlung auch für bestimmte Fächerwinkel (β) bestimmt werden. Aus dem Streustahlanteil für bestimmte Projektionswinkel α und Fächerwinkel β kann durch mathematische Methoden, wie z.B. eine geeignete Interpolation, durch Anpassung von modellbasierten Korrekturen an die gemessene Streustrahlwerte oder auch durch lernbasierte Verfahren die gesamte Streustrahlverteilung in α- und β- Richtung abgeschätzt werden.

Das erfindungsgemäße Verfahren wird vorzugsweise als Teil einer Bildaufnahme durchgeführt, wobei gleichzeitig Messwerte für die Ermittlung des Bildes und die Messwerte im Absorberschatten für die Ermittlung der Streustrahlverteilung gemessen werden können. Zum Beispiel können die Strahler bei der sequentiellen Aktivierung nacheinander in einem Durchgang des Messverfahrens aktiviert werden. Dabei können bei der Aktivierung eines Strahlers alle Detektoren innerhalb des Röntgenfächers Detektionswerte liefern, wobei die Detektionswerte innerhalb eines Absorberschattens Informationen über die Streustrahlverteilung liefern, und die Detektionswerte außerhalb des Absorberschattens Informationen für die Bildrekonstruktion liefern. Dabei werden die "abgeschatteten" Detektorelemente bei der Bildrekonstruktion z.B. durch geeignete Interpolation überbrückt. Bei der Bildrekonstruktion wird die Streustrahlverteilung entsprechend berücksichtigt. Dies hat den Vorteil, dass durch die Ermittlung der Streustrahlverteilung die Strahlenbelastung für den Patienten nicht steigt. Es ist möglich, dass bei der Aktivierung jedes Strahlers aus der Vielzahl von Strahlern während einer Bildaufnahme jeweils Messwerte in einem Absorberschatten gemessen werden und zur Ermittlung der Streustrahlverteilung beitragen. In diesem Fall umfasst die Untergruppe der Strahler alle Strahler aus der Vielzahl von Strahlern.

Alternativ kann das erfindungsgemäße Verfahren auch jeweils vor einer Bildaufnahme, insbesondere vor einer diagnostischen CT-Untersuchung, durchgeführt werden, wenn der Patient bereits im CT-Gerät positioniert wurde. Dadurch kann vorab für mehrere Bildaufnahmen die patientenspezifische Streustrahlverteilung gemessen werden.

Die Anzahl von Absorbern ist vorzugweise relativ klein im Vergleich zur Anzahl von Detektoren. Dies hat den Vorteil, dass die bei der Bildaufnahme akquirierten Daten nur geringfügig durch die Absorberschatten beeinträchtigt werden. Zum Beispiel können 4 bis 50, vorzugsweise 8 bis 32 Absorber vorgesehen sein. Die Anzahl der Absorber kann der Anzahl von Strahlern in der Untergruppe der Strahler entsprechen.

Ein Absorberschatten kann mittig und/oder seitlich innerhalb einer Röntgenfächers angeordnet sein. Dadurch können differenzierte Streustahleffekte untersucht werden.

Es ist denkbar, dass zur gleichen Zeit zwei Strahler der Untergruppe aktiviert werden, im Sinne eines "Dual Source" Betriebs. Dadurch können mehr Messwerte erlangt werden, als es mit nur einem singulär aktivierten Strahler möglich wäre. Dabei ist vorgesehen, dass die maximal zwei gleichzeitig emittierenden Strahler relativ zueinander einen Winkel von im Wesentlichen 90 Grad zum Mittelpunkt des Strahlerrings ausbilden. Folglich kann die von den Detektoren empfangene Strahlung zu dem Zeitpunkt der Detektion ihrem entsprechenden Ursprung zugeordnet werden. Dabei können beide Strahler um einen Winkel von 80 Grad bis 100 Grad, vorzugsweise 85 Grad bis 95 Grad, auf dem Strahlerring relativ zueinander beabstandet sein.

Vorzugsweise sind die Strahler der Untergruppe in zumindest ungefähr gleichen Winkelabständen um den Strahlerring verteilt. "Ungefähr" bedeutet hier mit einer Abweichung von +/-10%. Die Winkelabstände zwischen benachbarten Strahlern der Untergruppe können 360 Grad geteilt durch die Anzahl von Strahlern der Untergruppe entsprechen. Das liefert den Effekt, dass möglichst viele Winkelpositionen um das Objekt herum zur Bestimmung der Streustrahlverteilung herangezogen werden können.

Vorzugsweise umfasst die Untergruppe von Strahlern 0,1% bis 5%, vorzugsweise 0,2% bis 3%, besonders bevorzugt 0,4% bis 1,4% der Vielzahl von Strahlern. Der erste Bereich bietet den höchsten Informationsgehalt zur Auflösung der streustrahlverteilung. Dadurch können möglichst viele unterschiedliche Bereiche des Objektes auf deren Streu-, Reflektions- oder Emissionsverhalten untersucht werden, jedoch ist mehr Rechenleistung erforderlich. Im zweiten Bereich kann ein besonders ausgewogenes Verhältnis aus gesteigertem Informationsgehalt zur Auflösung der Streustrahlung und hoher Auflösung des erstellten CT-Bildes möglich sein. Der dritte Bereich kann einen ausreichenden Informationsgehalt zur Auflösung der Streustrahlung und eine gesteigerte Auflösung des CT-Bildes bieten. Mit anderen Worten kann der dritte Bereich eine maximale realitätsgetreue Abbildung des Objektes bereitstellen.

Vorzugsweise ist der Absorber dazu ausgestaltet, Röntgenstrahlung zu absorbieren, und enthält vorzugsweise Tantal oder Wolfram. Auf den Absorber auftreffende Röntgenstrahlung kann von diesem vollständig aufgenommen und insbesondere in Wärme umgewandelt werden. Vollständig bedeutet, dass ein Röntgenstrahl von einem Absorber unterbrochen werden kann und sich, insbesondere vollständig abgeschwächt, nicht fortsetzen kann. Wolfram oder Tantal können Bestandteile von Absorbern sein, da sie den Absorberschatten innerhalb des Röntgenfächers bewirken können.

Vorzugsweise erzeugt der Absorber einen absoluten Absorberschatten, welcher einen Teil des Strahlenfächers vollständig absorbiert. Der Absorber kann derart geometrisch ausgestaltet sein, dass er einen kleinen Anteil von 0% bis 5% des Fächerwinkels absorbiert. Der Absorber kann am Rand eines Objektschattens oder in der Mitte eines Objektschattens angeordnet sein. Dadurch können Detektionsergebnisse für die Streustrahlung an verschiedenen Fächerwinkeln erlangt werden.

Gemäß einer ersten Variante befindet sich der Absorber zwischen dem Strahler und dem Objekt, insbesondere in einem Abstand von 1 bis 10 cm vom Strahler. Mit anderen Worten kann sich der Absorberschatten durch das Objekt hindurch erstrecken. Vorteilhaft an dieser Ausführungsform ist, dass die Strahlenbelastung für den Patienten reduziert wird.

In einer Ausführungsform weist der Absorber eine gitterartige Struktur mit zumindest 2 Gitterelementen auf. Beispielsweise kann der Absorber zumindest einen Durchbruch aufweisen. Dadurch werden zumindest zwei voneinander getrennte Absorberschatten von nur einem Absorber erzeugt. Das hat den Vorteil, dass das von den in den zumindest zwei Absorberschatten gemessene Streusignal Aufschluss über den Verlauf der Streustrahlintensität in Fächerrichtung (β-Richtung) gibt.

In einer anderen Ausführungsform ist der Absorber ein sich in einer Axialrichtung des Strahlerrings erstreckender Stab, welcher eine Dicke von 0,1 mm bis 1 mm, vorzugsweise 0,1 mm bis 0,5 mm, besonders bevorzugt ungefähr 0,2 mm aufweist. Der Stab kann sich axial erstrecken, damit der Absorberschatten des Stabs den Detektorring erreichen kann, welcher von dem Strahlerring axial beabstandet ist. Es kann auch ein Gitter aus mehreren solcher Stäbe aufgebaut sein.

Gemäß einer zweiten Variante befindet sich der Absorber zwischen dem Objekt und den jeweils im Absorberschatten angeordneten Detektoren. Mit anderen Worten kann jene Strahlung absorbiert werden, welche bereits durch das Objekt hindurchgedrungen ist. Das hat den Vorteil, dass der vollständige Strahlenfächer mit dem Objekt wechselwirkt. Folglich kann eine maximale Streu- und Wechselwirkung mit dem Objekt erfolgen. Es können z.B. alle 30° bis 60°, z.B. alle 45° um den Detektorring verteilt Absorber angebracht werden.

In einer Ausführungsform der zweiten Variante ist der Absorber als Kollimatorblech ausgestaltet, welches zwischen zwei Detektorelementen angeordnet ist und sich zumindest im Wesentlichen radial in Richtung des Mittelpunkts des Detektorrings erstreckt. "Im Wesentlichen*"* ist so aufzufassen, dass die Erstreckung in Radialrichtung größer sein kann, als die Erstreckung in eine Richtung quer zur Radialrichtung. Je weiter sich das Kollimatorblech in die Radialrichtung erstreckt, desto größer kann der Absorberschatten sein. Mit anderen Worten ist die Anzahl an Detektoren im Absorberschatten abhängig von der Erstreckung des Kollimatorblechs in Radialrichtung.

In einer anderen Ausführungsform der zweiten Variante ist der Absorber als Kollimatorblech ausgestaltet, welches vom Detektorring beabstandet ist und sich im Wesentlichen senkrecht zur Radialrichtung des Detektorrings erstreckt. "Im Wesentlich*"* ist so aufzufassen, dass die Erstreckung in eine Richtung senkrecht zur Radialrichtung größer sein kann, als die Erstreckung in die Radialrichtung.

Vorzugsweise wird die Streustrahlverteilung durch mathematische Verfahren, insbesondere Interpolation der Messwerte, und/oder Anpassung modellbasierter Korrekturen an die erfassten Messwerte oder durch lernbasierte Verfahren, bestimmt. Eine Streustrahlverteilung kann eine Beaufschlagung von der jeweiligen Streustrahlung auf ein jedes Detektorelement umfassen. Da nur ein Teil der Vielzahl von Detektorelementen die Streustrahlung direkt misst, werden vorzugsweise die Streustrahlwerte an den restlichen Detektorelementen mathematisch berechnet, insbesondere geschätzt. Die Streustrahlung der restlichen Detektorelemente kann basierend auf den gemessenen Streustrahlwerten der anderen Detektorelementen, insbesondere durch Interpolation, berechnet werden. Hierfür kann eine kontinuierliche Funktion entwickelt werden, die diskrete Stützwerte, insbesondere Strahlenwerte, interpoliert. Das kann den Effekt liefern, dass eine kontinuierliche Bestimmung der Streustrahlverteilung an jedem Punkt des Detektorrings möglich sein kann. Alternativ kann die Streustrahlverteilung durch lernbasierte Verfahren aus den einzelnen Stützwerten bestimmt werden.

Vorzugsweise wird das Verfahren vor oder während der Aufnahme eines Computertomographiebildes durchgeführt, wobei das Computertomographiebild um die bestimmte Streustrahlverteilung des Objektes korrigiert wird. Mit anderen Worten kann zunächst die Streustrahlverteilung als vorangestellter Schritt einer CT-Aufnahme durchgeführt werden. Entsprechende Messwerte, die für die Berechnung des CT-Bildes detektiert werden, können dann vor der mathematischen Zusammensetzung zur Berechnung des CT-Bildes um die ermittelte Streustrahlung für jeden einzelnen der Vielzahl an Detektoren korrigiert werden. Genauer gesagt, kann insbesondere von den Messwerten eines jeweiligen Detektors eine absolute Streustrahlgröße abgezogen werden, welche durch die Streustrahlverteilung bestimmt ist. Dadurch kann die Korrektur eines CT-Bildes bereits erfolgen, bevor das CT-Bild selbst berechnet ist.

Ein weiterer Aspekt der Erfindung betrifft ein Computertomographiegerät (CT-Gerät), welches zur Ausführung des Verfahrens konfiguriert ist, umfassend einen stationären Detektorring und einen stationären Strahlerring, wobei der Detektorring eine Vielzahl von Detektoren und der Strahlerring eine Vielzahl von Strahlern umfasst, wobei der Strahlerring eine Untergruppe aus der Vielzahl von Strahlern aufweist, welche zur Ermittlung der Streustrahlverteilung einzeln oder paarweise aktivierbar sind, wobei je Strahler ein Strahlenfächer erzeugbar ist, wobei das Computertomographiegerät eine Mehrzahl von Absorbern aufweist, welche innerhalb eines Strahlenfächers der Untergruppe von Strahlern angeordnet sind, wobei ein Absorberschatten erzeugbar ist, wobei der Detektorring Detektoren aufweist, welche dazu konfiguriert sind, Messwerte im Absorberschatten zu erfassen, und eine Recheneinheit zur Verarbeitung der erfassten Messwerte, wobei eine Streustrahlverteilung auf Basis der erfassten Messwerte berechnet wird.

Alle Merkmale und Vorteile des erfindungsgemäßen Verfahrens finden auch auf das CT-Gerät Anwendung und umgekehrt.

Ein Computertomographiegerät oder ein Computertomograph können eine Vorrichtung zur Ausführung eines bildgebenden Verfahrens sein. Das Computertomographiegerat ist dazu ausgestaltet, CT-Bilder von Objekten zu erstellen, wobei die CT-Bilder um eine Streustrahlung korrigiert werden können. Durch Streustrahlung kann es zu einer Abweichung von einer wahrheitsgemäßen Darstellung eines Objekts kommen. Mit anderen Worten kann die Vorrichtung derart konfiguriert sein, dass eine möglichst wahrheitsgetreue Abbildung des Objektes berechnet werden kann, insbesondere, dass Artefakte korrigiert sind. Dazu weist die Vorrichtung einen stationären Detektorring und einen stationären Strahlerring auf, wobei beide Elemente konzentrisch zueinander und voneinander axial beabstandet angeordnet sind. Dadurch kann Strahlung, insbesondere Röntgenstrahlung, von jeder umfänglichen Position des Strahlerrings auf jede umfängliche Stelle auf den Detektorring treffen. Der Detektorring umfasst eine Vielzahl von Detektoren, welche insbesondere mit gleichbleibenden Abständen umfänglich an dem Detektorring angeordnet sind. Ferner umfasst der Strahlerring eine Vielzahl an Strahlern, welche umfänglich an dem Strahlerring angeordnet sind. Jeder der Strahler des Strahlerrings ist dazu ausgestaltet, Strahlung in Form eines Strahlenfächers zu emittieren. Der Strahlenfächer kann durch einen Fächerwinkel beschrieben werden. Eine Untergruppe von der Vielzahl von Strahlern kann gebildet werden, wobei jeder der Strahler der Untergruppe von Strahlern dazu verwendet wird, um die Streustrahlverteilung für eine Aufnahme des Objektes innerhalb des CT zu bestimmen. Die Strahler der Untergruppe von Strahlern sind dabei einzeln oder paarweise aktivierbar. Mit anderen Worten können zu einem Zeitpunkt immer nur ein Strahler oder maximal zwei Strahler gleichzeitig emittieren. Insbesondere können die Strahler sequentiell aktiviert werden. Das kann den Effekt liefern, dass mehr Messwerte erlangt werden, als es mit nur einem singulär aktivierten Strahler möglich wäre. Vorzugsweise kann vorgesehen sein, dass die maximal zwei gleichzeitig emittierenden Strahler relativ zueinander einen Winkel von im Wesentlichen 90 Grad zum Mittelpunkt des Strahlerrings ausbilden. Folglich kann die von den Detektoren empfangene Strahlung zu dem Zeitpunkt der Detektion ihres entsprechenden Ursprungs zugeordnet werden. Die Strahlenfächer der Untergruppe von Strahlern können auf den Detektorring treffen. Darüber hinaus sind ein oder mehrere Absorber in die Strahlenfächer der Untergruppe von Strahlern positionierbar. Diese Absorber bewirken einen Absorberschatten, welcher auf jene oder nur auf einen Teil von jenen Detektoren fällt, die innerhalb eines jeweiligen Strahlenfächers der Untergruppe von Strahlern liegen. Mit anderen Worten fallen diese Absorberschatten auf einzelne Detektorelemente von der Vielzahl an Detektorelementen. Deren Messwerte können als Stützwerte zur Bestimmung der Streustrahlverteilung herangezogen werden, da man davon ausgeht, dass die in den Absorberschatten gemessene Strahlung Streustrahlung sein muss. Zusätzlich umfasst die Vorrichtung der Erfindung eine Recheneinheit oder einen Computer zur Verarbeitung der erfassten Messwerte und zur Berechnung der Streustrahlverteilung. Ferner kann die Recheneinheit gemessene Strahlungen für jeden der einzelnen Detektoren um die ermittelte Streustrahlverteilung korrigieren. Folglich kann das CT-Bild auf Basis korrigierter Messwerte ermittelt werden. Insbesondere können die Messwerte zur Berechnung des CT-Bildes an jenen Stellen interpoliert werden, an welchen die Detektoren im Absorberschatten liegen. Mit anderen Worten jene Detektoren, welche zur Ermittlung der Streustrahlverteilung verwendet werden, können zur Berechnung des CT-Bildes interpoliert, insbesondere approximiert werden. Das kann den Effekt liefern, dass die Auflösung eines CT-Bildes gesteigert wird. In einer bevorzugten Ausführungsform handelt es sich bei dem CT der vorliegenden Erfindung um ein statisches CT. Das bedeutet, dass sowohl der stationäre Detektorring als auch der stationäre Strahlerring nicht beweglich, insbesondere nicht relativ zueinander drehbar, angeordnet sind. Folglich bleiben Relativpositionen zwischen einem der Detektoren und einem der Strahler konstant. Der Detektorring und der Strahlerring sind insbesondere zueinander konzentrisch. Ferner erstreckt sich eine Längsachse durch und über die Mittelpunkte des kreisförmigen Detektor- respektive kreisförmigen Strahlerrings hinaus. Entlang der Längsachse können der Detektorring und der Strahlerring zueinander beabstandet sein. Dadurch kann Röntgenstrahlung vollumfänglich des Strahlerrings emittiert erst durch das Objekt und dann auf den Detektorring treffen. An dem Detektorring ist eine Vielzahl von Detektoren, insbesondere zueinander benachbart, angeordnet. Der Strahlerring umfasst eine Vielzahl von Strahlern, welche dazu ausgestaltet sind, unabhängig voneinander Röntgenstrahlung gesteuert zu emittieren. Dadurch können der Ursprung von Röntgenstrahlung und somit der Projektionswinkel steuerbar sein. Genauer gesagt umfasst insbesondere jeder Strahler eine dazugehörige Kathode, welche steuerbar ist. Von der steuerbaren Kathode können je Strahler Elektronen emittiert und hin zu einer Anode unter einer Spannung beschleunigt werden, welche unter Aussendung einer Bremsstrahlung bzw. Röntgenstrahlung an der Anode abgebremst werden. Die Kathoden sind insbesondere zueinander winkelversetzt und vorzugsweise gleichmäßig über den Umfang des Strahlerrings hinweg verteilt angeordnet. Von der Vielzahl von Strahlern wird eine Untergruppe gebildet, wobei die Strahler der Untergruppe zur Ermittlung der Streustrahlverteilung verwendet werden, insbesondere auch zum Informationsgewinn des CT-Bildes beitragen. Der Röntgenfächer eines Strahlers ist insbesondere durch den Fächerwinkel, in einer Ebene parallel zu dem Detektorring oder Strahlerring, bestimmt. Der Röntgenfächer kann radial, ausgehend von einem der Strahler, expandieren. Ein Absorberschatten entsteht, indem ein oder mehrere Absorber in den Röntgenfächer gebracht werden, welche die Röntgenstrahlung teilweise, vorzugsweise vollständig, absorbieren. Unter einer vollständigen Absorption ist eine vollständige Aufnahme ohne weitere Reflektion oder Emission der Röntgenstrahlung zu verstehen. In dem folglich entstehenden Absorberschatten können Detektoren des Detektorrings umfasst sein. Durch die Geometrie des Absorbers kann die räumliche Ausdehnung des Absorberschattens vorbestimmt werden. Folglich können sowohl einzelne als auch mehrere Detektoren innerhalb des Absorberschattens liegen. Das kann den Effekt liefern, dass der erlangte Informationsgehalt gesteigert und zusätzlich diversifiziert ist. Ausgehend von der gemessenen Streustrahlung, die ursprünglich von der Untergruppe von Strahlern vor dem zumindest einem Absorber gemessen wurde, können Interpolationswerte über eine Streustrahlbeaufschlagung der Vielzahl von Detektoren des Detektorrings auf der Recheneinheit errechnet werden. Diese jeweils bestimmte Streustrahlbeaufschlagung wird auch als Streustrahlverteilung bezeichnet.

Insbesondere kann die Mehrzahl von Absorbern an festgelegten Positionen am Computertomographiegerät festgelegt sein. Die Absorber können vorzugsweise an einem ringförmigen Trägerelement angeordnet sein, dessen Durchmesser kleiner als der Durchmesser des Strahlerrings ist. Dadurch kann vom Strahlerring ausgehende Röntgenstrahlung auf zumindest einen Absorber treffen, welcher in Strahlenrichtung vor einem Detektor angeordnet ist. Insbesondere können die Absorber herausnehmbar an dem Trägerelement angeordnet sein. Das kann die Zugänglichkeit bei Wartungsarbeiten steigern. Zusätzlich kann die Position von Absorbern vor oder nach einer Messung variiert werden. Ferner kann das Trägerelement eine Vielzahl an Arretierungselementen aufweisen, welche ausgestaltet sind, um einen Absorber positionsfest zu fixieren. Die Arretierungselemente können dabei in regelmäßigen Abständen zueinander beabstandet sein. Folglich kann ein ganzzahliges Vielfaches eines Absorbers an dem Trägerelement angeordnet werden.

Einzelne Ausführungsformen und Merkmale können mit anderen Ausführungsformen und anderen Merkmalen kombiniert werden und so neue Ausführungsformen bilden. Ausgestaltungen und Vorteile der Ausführungsformen und der Merkmale gelten analog auf für die neuen Ausführungsformen. Ferner gelten Ausgestaltungen und Vorteile, die in Verbindung mit der Vorrichtung genannt wurden analog auf für das Verfahren und andersherum.

Im Folgenden werden Ausführungsformen der vorliegenden Erfindung unter Bezugnahme auf die beigefügten Figuren im Detail beschrieben. Dabei zeigen:
- **Fig. 1:**: ein Computertomographiegerät und Recheneinheit gemäß einer Ausführungsform der vorliegenden Erfindung,
- **Fig. 2:**: ein Computertomographiegerät gemäß einer Ausführungsform der vorliegenden Erfindung, wobei der Absorber zwischen Strahlenring und Objekt angeordnet ist,
- **Fig. 3:**: ein Computertomographiegerät gemäß einer Ausführungsform der vorliegenden Erfindung, wobei der Absorber als Kollimator zwischen benachbarten Detektoren angeordnet ist,
- **Fig. 4:**: ein Ablaufdiagramm zum Bestimmen einer Streustrahlverteilung.

In den Figuren sind gleiche Merkmale mit den gleichen Bezugszeigen gekennzeichnet.

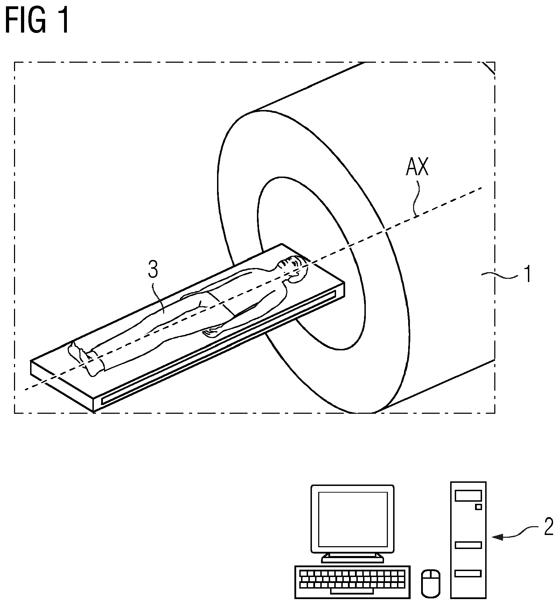

Fig. 1 ist eine Darstellung eines Computertomographiegerätes 1 gemäß einer Ausführungsform der Erfindung, welches derart ausgestaltet ist, dass ein Objekt 3 in das Computertomographiegerät 1 entlang einer Axialrichtung AX hineingeschoben werden kann. Als Objekt 3 ist in der Fig. 1 ein Mensch oder ein Patient dargestellt, welcher liegend vor dem Computertomographiegerät 1 positioniert ist. Des Weiteren ist eine Recheneinheit 2 in Form eines Computers dargestellt. Die Recheneinheit 2 kann ein Teil des Computertomographiegerätes 1 sein, insbesondere mit dem Computertomographiegerät 1 kommunizieren und Daten, beispielsweise erfasste Messwerte, austauschen.

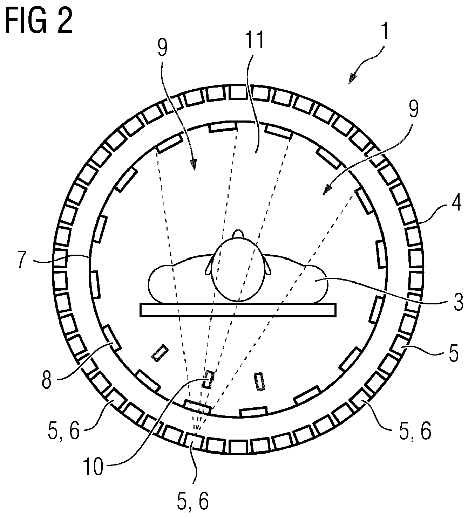

Fig. 2 zeigt einen Querschnitt eines Computertomographiegerätes 1 gemäß einer Ausführungsform der Erfindung. Das Objekt 3, insbesondere der Mensch oder Patient, ist mittig innerhalb des Detektorrings liegend platziert. Der Detektorring selbst ist ein runder vollumfänglicher Kreis, auf dem eine Vielzahl von Detektoren 8 in einem zueinander gleichmäßigen Abstand angeordnet sind. Die Detektoren 8 sind dazu ausgestaltet, Röntgenstrahlung zu detektieren, die von einer Vielzahl von Strahlern 5 ausgeht. Die Strahler sind wiederum an einem Strahlerring angeordnet, welcher konzentrisch zu dem Detektorring 7 orientiert ist, wobei der Strahlerring 4 einen größeren Durchmesser als der Detektorring 7 aufweist. Die Axialrichtung AX erstreckt sich durch die Mittelpunkte des Strahlerrings 4 und des Detektorrings 7 hindurch. Die Winkelposition eines Strahlers 5 auf dem Strahlerring 4 wird als Projektionswinkel (β) bezeichnet. Ein Strahler 5 ist dazu ausgestaltet, Röntgenstrahlung in Form eines sich aufweitenden Strahlenfächers 9 zu emittieren. Der Winkel innerhalb des Strahlenfächers ist durch den Fächerwinkel (α) beschrieben.

Die Strahler 5 werden so angesteuert, dass sie sequentiell emittieren, wobei zu einem Zeitpunkt nicht mehr als zwei Strahler 5 gleichzeitig emittieren. Sequentiell kann bedeuten, dass ein Strahler 5 nach dem anderen Strahler 5 emittiert, wobei die Reihenfolge der Strahler 5 variieren kann und in der Sequenz einzelne Strahler ausgelassen werden können. Damit kann die Strahlenbelastung für das Objekt, insbesondere den Patienten, reduziert werden. Von der Vielzahl von Strahlern 5 wird eine Untergruppe von Strahlern 6 gebildet. Die Untergruppe von Strahlern 6 kann 0,1% bis 5% der Vielzahl von Strahlern 5 umfassen. Beispielsweise kann die Untergruppe an Strahler 4 bis 50, vorzugsweise 8 bis 32, z.B. 20 Strahler umfassen, die in einem mittleren Winkelabstand von 360°/20=18° angeordnet sind. Ferner umfasst die Untergruppe von Strahlern 6 jene Strahler 5, welche sowohl zum Informationsgewinn für die mathematische Berechnung eines CT-Bildes, als auch für die Ermittlung der Streustrahlverteilung verwendet werden. Die Untergruppe von Strahlern 6 hat die Eigenschaft, dass von den darin umfassten Strahlern 5 ein Strahlenfächer 9 ausgehen kann, welcher auf einen Absorber 10 treffen. Trifft ein Röntgenstrahl des Strahlenfächers 9 auf einen Absorber, so wird dieser von dem Absorber abgeschwächt, insbesondere vollständig absorbiert. Mit anderen Worten kann sich ein Röntgenstrahl in Strahlenrichtung nach dem Absorber 10 nicht weiter fortsetzen. Der Bereich in Strahlenrichtung hinter einem Absorber 10, der sich innerhalb eines Strahlenfächers 9 von einem Strahler 5 der Untergruppe von Strahlern 6 erstreckt, ist als Absorberschatten 11 dargestellt. Da sowohl der Strahlenfächer 9, als auch der Absorberschatten 11 den Detektorring erreichen, können Detektoren 8 sowohl innerhalb, als auch außerhalb des Absorberschattens 11 angeordnet sein. Weiter zeigt Fig. 2 mehrere schematisch dargestellte Absorber 10, die innerhalb des Detektorrings 7, insbesondere voneinander in gleichmäßigen Abständen, angeordnet sind. Insbesondere kann eine größere Anzahl als die in Fig. 2 dargestellten Absorber in einer konsekutiven Fortsetzung der dargestellten Absorber angeordnet sein. Ferner sind die Absorber 10 so angeordnet, dass ein davon ausgehender Absorberschatten 11 zunächst auf das Objekt 3 trifft. Diese Anordnung hat den Vorteil, dass die Strahlenbelastung für den Patienten reduziert wird. Gemäß Fig. 2 sind die Absorber 10 als dünne Metallstäbe dargestellt, die eine Breite in lateraler Richtung von 0,2 mm aufweisen. Der davon ausgehende Absorberschatten 11 kann in einer Entfernung von etwa 1 m auf den Detektorring 7 treffen, wobei dort ein Bereich von ungefähr 2,0 mm vom Absorberschatten 11 belegt ist.

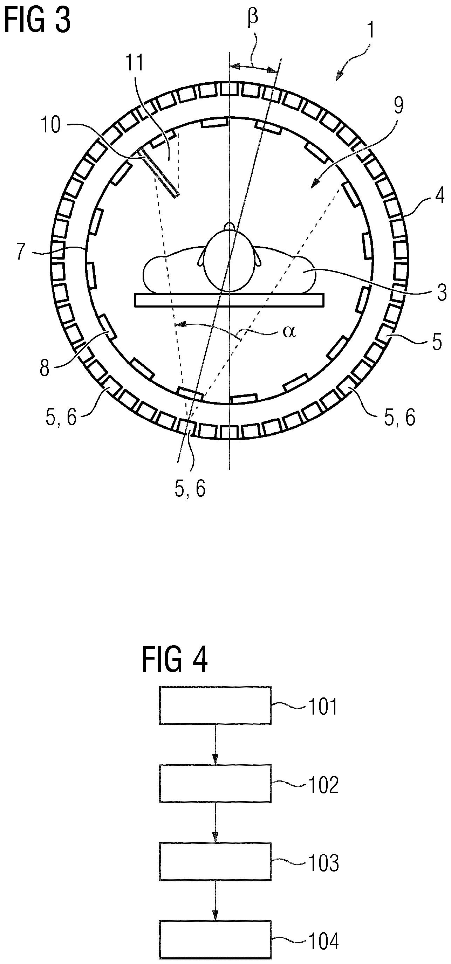

Fig. 3 zeigt einen vergleichbaren Aufbau des Computertomographiegeräts 1, wie es in Fig. 2 gezeigt ist. Jedoch unterscheidet sich der Gegenstand der Fig. 3 maßgeblich in der Ausgestaltung und Anordnung der Absorber 10. Fig. 3 zeigt einen Absorber 10, welcher in Strahlenrichtung nach dem Objekt 3 zwischen zwei Detektoren 8 angeordnet ist. Der Absorber 10 kann insbesondere als ein Kollimatorblech ausgestaltet sein. Der Absorber 10 erstreckt sich vorwiegend radial in Richtung des Mittelpunktes des Detektorrings 7. Innerhalb des Absorberschattens 11 kann ein Detektor 8 angeordnet sein. Ähnlich wie bei dem Gegenstand der Fig. 2 kann dieser Detektor 8 Streustrahlung messen und Informationen zur Streustrahlverteilung liefern. Ferner können auch mehrere als Kollimatorblech ausgestaltete Absorber 10 zwischen weitere benachbarte Detektoren 8 des Detektorrings 7 angeordnet sein und somit weitere Absorberschatten 11 erzeugen. Eine exemplarische Darstellung eines Projektionswinkels (β) und eines Fächerwinkels (α) ist in Fig. 3 dargestellt.

Fig. 4 ist ein Ablaufdiagramm, das die Prozessschritte eines Verfahrens gemäß einer Ausführungsform der Erfindung darstellt. Der erste Schritt umfasst eine sequenzielle Aktivierung der Strahler 101. In diesem Schritt wird die Vielzahl von Strahlern 5 nacheinander, aber zu einem Zeitpunkt maximal zwei Strahler 5 gleichzeitig, aktiviert, wobei die Röntgenstrahlung in Form des Strahlenfächers 9 von den Strahlern 5 emittiert wird. Von der Vielzahl von Strahlern 5 ist eine Untergruppe von Strahlern 6 abzutrennen, welche vorwiegend zur Berechnung der Streustrahlverteilung verwendet werden. Im darauffolgenden Schritt kommt es zu einer Erzeugung des Absorberschattens 102, wobei der Strahlenfächer, insbesondere ein Ausschnitt oder Teil des Strahlenfächers, auf einen oder mehrere Absorber trifft, sodass der Strahlenfächer in diesen Bereichen abgeschwächt, insbesondere vollständig absorbiert wird. Der Strahlenfächer 9 des zweiten Verfahrensschrittes geht insbesondere von der Untergruppe von Strahlern 6 aus. Dabei erfolgt eine Detektion der Röntgenstrahlung 103, wobei derjenige Teil des Strahlenfächers 9 auf in Strahlenrichtung dahinterliegende Detektoren 8 trifft und gemessen wird. Innerhalb dieses Schrittes werden auch Strahlenintensitäten innerhalb des Absorberschattens 11 detektiert. Diese Intensitäten weisen auf eine Streustrahlung hin und werden im folgenden Schritt zur Berechnung der Streustrahlverteilung 104 herangezogen. Beispielswese können die Messwerte der Streustrahlung an einzelnen Detektoren 8 für unterschiedliche Projektionswinkel ermittelt werden und als Stützwerte für eine Interpolation herangezogen werden, die eine vollumfängliche Streustrahlverteilung für jeden Detektor bestimmen kann. Insbesondere ist die Streustrahlverteilung ein Absoluter Strahlenwert, welcher spezifisch für ein Objekt 3 für jeden der Vielzahl von Detektoren 8 bestimmt werden kann.

## Patentansprüche

1. Verfahren zum Bestimmen einer Streustrahlverteilung bei einem Objekt (3), welches in einem Computertomographiegerät (1) mit einem stationären Detektorring (7) und einem stationären Strahlerring (4) angeordnet ist, wobei der Detektorring (7) eine Vielzahl von Detektoren (8) und der Strahlerring (4) eine Vielzahl von Strahlern (5) umfasst, umfassend die Schritte
sequenzielle Aktivierung (101) von jeweils einem oder jeweils zwei Strahlern (5) aus einer Untergruppe der Vielzahl von Strahlern (6) des Strahlerrings (4), zur Erzeugung von jeweils einem oder jeweils zwei Strahlenfächern (9) aus Röntgenstrahlung,
Erzeugung eines Absorberschattens (102) innerhalb des jeweils erzeugten Strahlenfächers (9) durch zumindest einen im Strahlenfächer (9) angeordneten Absorber (10),
Detektion der Röntgenstrahlung (103) im Absorberschatten (11) durch die jeweils im Absorberschatten (11) angeordneten Detektoren (8) des Detektorrings (9) und dadurch Erfassung von Messwerten,
Berechnung der Streustrahlverteilung (104) auf Basis der erfassten Messwerte.

2. Verfahren nach Anspruch 1, wobei die Strahler der Untergruppe (6) in zumindest ungefähr gleichen Winkelabständen um den Strahlerring (4) verteilt sind.

3. Verfahren nach Anspruch 1 oder 2, wobei die Untergruppe von Strahlern (6) 0,1% bis 5%, vorzugsweise 0,2% bis 3%, besonders bevorzugt 0,4% bis 1,5% der Vielzahl von Strahler (5) umfasst.

4. Verfahren nach einem der vorherigen Ansprüche, wobei beide Strahler (5) um einen Winkel von 80° bis 100°, vorzugsweise 85° bis 95°, auf dem Strahlerring (4) relativ zueinander beabstandet sind, insbesondere wobei ein Projektionswinkel des einen Strahlers (5) von dem Projektionswinkel des zweiten Strahlers (5) um 80° bis 100°, vorzugsweise 85° bis 95°abweicht.

5. Verfahren nach einem der vorherigen Ansprüche, wobei der Absorber (10) dazu ausgestaltet ist, Röntgenstrahlung zu absorbieren, und vorzugsweise Tantal oder Wolfram umfasst.

6. Verfahren nach einem der vorherigen Ansprühe, wobei der Absorber (10) einen absoluten Absorberschatten (11) erzeugt, welcher einen Teil des Strahlenfächers (9) vollständig absorbiert.

7. Verfahren nach einem der vorherigen Ansprüche, wobei sich der Absorber (10) zwischen dem Strahler (5) und dem Objekt (3) befindet, insbesondere in einem Abstand von 1 bis 10 cm vom Strahler (5).

8. Verfahren nach einem der vorherigen Ansprüche 1-7, wobei der Absorber (11) ein sich in einer Axialrichtung (AX) des Strahlerrings (4) erstreckender Stab ist, welcher eine Dicke von 0,1 mm bis 1 mm, vorzugsweise 0,1 mm bis 0,5 mm, besonders bevorzugt 0,2 mm aufweist.

9. Verfahren nach einem der vorherigen Ansprüche 1-8, wobei sich der Absorber (10) zwischen dem Objekt (3) und den jeweils im Absorberschatten (11) angeordneten Detektoren (8) befindet.

10. Verfahren nach Anspruch 9, wobei der Absorber (10) als Kollimatorblech ausgestaltet ist, welches zwischen zwei Detektorelementen (8) angeordnet ist und sich zumindest im Wesentlichen radial in Richtung des Mittelpunkts des Detektorrings (7) erstreckt

11. Verfahren nach einem der vorherigen Ansprüche, wobei die Streustrahlverteilung durch mathematische Verfahren, insbesondere Interpolation der Messwerte, und/oder Anpassung modellbasierter Korrekturen an die erfassten Messwerte oder durch lernbasierte Verfahren, bestimmt wird.

12. Verfahren nach einem der vorherigen Ansprüche, welches vor oder während der Aufnahme eines Computertomographiebildes durchgeführt wird, wobei das Computertomographiebild um die bestimmte Streustrahlverteilung des Objektes korrigiert wird.

13. Computertomographiegerät, welches zur Ausführung des Verfahrens nach einem der Ansprüche 1-12 konfiguriert ist, umfassend einen stationären Detektorring (7) und einen stationären Strahlerring (4), wobei der Detektorring (7) eine Vielzahl von Detektoren (8) und der Strahlerring (4) eine Vielzahl von Strahlern (5) umfasst,
wobei der Strahlerring (4) eine Untergruppe aus der Vielzahl von Strahlern (6) aufweist, welche zur Ermittlung der Streustrahlverteilung einzeln oder paarweise aktivierbar sind, wobei je Strahler (5) ein Strahlenfächer (9) erzeugbar ist,
wobei das Computertomographiegerät (1) eine Mehrzahl von Absorbern (10) aufweist, welche innerhalb eines Strahlenfächers (9) der Untergruppe von Strahlern (9) angeordnet sind, wobei ein Absorberschatten (11) erzeugbar ist,
wobei der Detektorring (7) Detektoren (8) aufweist, welche dazu konfiguriert sind, Messwerte im Absorberschatten (11) zu erfassen, und
umfassend eine Recheneinheit (2) zur Verarbeitung der erfassten Messwerte, welche dazu konfiguriert ist, eine Streustrahlverteilung auf Basis der erfassten Messwerte zu berechnen.

14. Computertomographiegerät gemäß Anspruch 13, wobei die Mehrzahl von Absorbern (10) an festgelegten Positionen am Computertomographiegerät (1) angeordnet ist.
